(19) Europäisches Patentamt
European Patent Office
Office européen des brevets

(11) **EP 2 163 190 A1**

(12) **EUROPÄISCHE PATENTANMELDUNG**

(43) Veröffentlichungstag:
**17.03.2010 Patentblatt 2010/11**

(51) Int Cl.:
***A61B 5/00*** *(2006.01)*

(21) Anmeldenummer: **08015982.5**

(22) Anmeldetag: **11.09.2008**

(84) Benannte Vertragsstaaten:
**AT BE BG CH CY CZ DE DK EE ES FI FR GB GR HR HU IE IS IT LI LT LU LV MC MT NL NO PL PT RO SE SI SK TR**
Benannte Erstreckungsstaaten:
**AL BA MK RS**

(71) Anmelder:
• **Roche Diagnostics GmbH**
**68305 Mannheim (DE)**
Benannte Vertragsstaaten:
**DE**
• **F.HOFFMANN-LA ROCHE AG**
**4070 Basel (CH)**
Benannte Vertragsstaaten:
**AT BE BG CH CY CZ DK EE ES FI FR GB GR HR HU IE IS IT LI LT LU LV MC MT NL NO PL PT RO SE SI SK TR**

(72) Erfinder:
• **Quarder, Ortrud**
**69115 Heidelberg (DE)**
• **Mischler, Reinhold**
**67063 Ludwigshafen (DE)**
• **Rieger, Ewald**
**67240 Bobenheim-Roxheim (DE)**
• **Staib, Arnulf**
**64646 Heppenheim (DE)**
• **Gillen, Ralph**
**26871 Papenburg (DE)**
• **Kamecke, Ulrike**
**68167 Mannheim (DE)**

(74) Vertreter: **Twelmeier Mommer & Partner**
**Patent- und Rechtsanwälte**
**Westliche 56-68**
**75172 Pforzheim (DE)**

(54) **Elektrodensystem für Messung einer Analytkonzentration in-vivo**

(57)     Die Erfindung geht aus von einem Elektrodensystem zur in-vivo Messung der Konzentration eines Analyten, umfassend eine Gegenelektrode (2) mit einem elektrischen Leiter (2a), eine Arbeitselektrode (1) mit einem elektrischen Leiter (1a), auf dem eine Enzymschicht (5) angeordnet ist, die immobilisierte Enzymmoleküle zur katalytischen Umwandlung des Analyten enthält, und einer Diffusionsbremse, welche die Diffusion des Analyten aus das Elektrodensystem umgebender Körperflüssigkeit zu Enzymmolekülen verlangsamt.

Erfindungsgemäß ist vorgesehen, dass die Enzymschicht (5) in Form von mehreren Feldern ausgebildet ist, die auf dem Leiter (1a) der Arbeitselektrode (1) in einem Abstand von einander angeordnet sind.

Fig. 1

EP 2 163 190 A1

**Beschreibung**

Die Erfindung betrifft ein Elektrodensystem zur Messung einer Analytkonzentration in-vivo mit den im Oberbegriff des Anspruchs 1 angegebenen Merkmalen. Ein derartiges Elektrodensystem ist aus der WO 2007/147475 bekannt.

Sensoren mit implantierbaren oder insertierbaren Elektrodensystemen ermöglichen Messungen von physiologisch bedeutsamen Analyten wie beispielsweise Laktat oder Glukose im Körpergewebe eines Patienten. Die Arbeitselektroden derartiger Systeme weisen elektrisch leitfähige Enzymschichten auf, in denen Enzymmoleküle gebunden sind, die durch katalytische Umwandlung von Analytmolekülen Ladungsträger freisetzten. Dabei wird als Messsignal ein elektrischer Strom erzeugt, dessen Stärke mit der Analytkonzentration korreliert.

Gute Enzymschichten sollten eine möglichst hohe elektrisch Leitfähigkeit aufweisen, damit freigesetzte Ladungsträger möglichst vollständig als Messsignal erfasst werden können, in ausreichendem Maße wasserdurchlässig sein, damit Analytmoleküle aus wässriger Körperflüssigkeit, in der Regel interstitieller Flüssigkeit oder Blut, in die Enzymschicht diffundieren können, und schließlich enthaltene Enzymmoleküle möglichst vollständig binden, so dass diese nicht in umgebendes Körpergewebe austreten können.

Geeignete Enzymschichten können beispielsweise aus Platinschwarz, das mit Enzymlösung imprägniert werden kann und wegen seiner schwammartigen Struktur gut wasserdurchlässig ist, oder aus elektrisch leitfähigen Partikeln, beispielsweise Kohlenstoffpartikeln, und einem Bindemittel hergestellt werden. Derartige Enzymschichten sind in der Regel spröde. Bei bekannten Sensoren bedeckt die Enzymschicht der Arbeitselektrode deshalb nur eine sehr kleine Fläche, in Regel nur einen Bruchteil eines Quadratmillimeters. Ein Beispiel hierfür ist das aus der US 4,655,880 bekannte Elektrodensystem, bei dem sich der Leiter der Arbeitselektrode nur über etwa 200 $\mu$m erstreckt. Zur Erhöhung der lokalen Stromdichte ist dieser Leiter mit einem elektrisch isolierenden Überzug versehen, in den kleine Fenster mit einem Durchmesser von etwa 10 $\mu$m geätzt wurden, bevor der Leiter zur Ausbildung einer Enzymschicht auf seiner Gesamtlänge mit einer enzymhaltigen Paste überzogen wurde.

Trotz intensiver Forschungs- und Entwicklungsarbeiten sind bekannte Elektrodensysteme jedoch störanfällig und haben den Nachteil, dass sich mit ihnen Analytkonzentration nur mit einer geringeren Genauigkeit und Zuverlässigkeit bestimmen lassen, als es mit einer herkömmlichen ex-vivo Analyse möglich ist.

Zur Erhöhung der Messgenauigkeit wird in der US 2005/0059871 A1 vorgeschlagen, die interessierende Analytkonzentration mit mehreren Arbeitselektroden gleichzeitig zu messen und die so erhaltenen Messungen statistisch auszuwerten. Als zusätzliche Maßnahme wird empfohlen, ergänzend mit weiteren Sensoren andere Analytkonzentration oder physiologisch Parameter zu bestimmen und anhand der ermittelten Konzentration von verschiedenen Analyten eine Plausibilitätsprüfung der einzelnen Resultate durchzuführen.

Die Verwendung einer großen Zahl von Arbeitselektroden erhöht jedoch einerseits den apparativen Aufwand und führt andererseits zu dem Problem, dass bei widersprüchlichen Messsignalen der einzelnen Arbeitselektroden unklar ist, welche der verschiedenen Messwerte die Analytkonzentration im Körper des Patienten zutreffend wiedergeben.

Aufgabe der Erfindung ist es deshalb, einen Weg aufzuzeigen, wie Analytkonzentrationen mit größerer Zuverlässigkeit und Genauigkeit in einem menschlichen oder tierischen Körper gemessen werden können.

Diese Aufgabe wird durch ein Elektrodensystem zur in-vivo Messung der Konzentration eines Analyten mit den im Anspruch 1 angegebenen Merkmalen gelöst. Vorteilhafte Weiterbildungen der Erfindung sind Gegenstand der Unteransprüche.

Erfindungsgemäß ist vorgesehen, dass die Enzymschicht auf der Arbeitselektrode in Form von mehreren Feldern ausgebildet ist, die auf dem Leiter der Arbeitselektrode in einem Abstand voneinander angeordnet sind. Bevorzugt sind mindestens zwei dieser Felder voneinander mindestens drei Millimeter, vorzugsweise mindestens fünf Millimeter, entfernt. Beispielsweise kann eine Folge von mehreren Feldern vorgesehen sein, wobei der Abstand zwischen dem ersten und dem letzten Feld der Folge mehr als fünf Millimeter beträgt. Die einzelnen Felder einer erfindungsgemäßen Arbeitselektrode bilden gewissermaßen eine Folge von in Reihe geschalteten Arbeitselektroden.

Die erfindungsgemäße Maßnahme ermöglicht wesentlich zuverlässigere Messungen einer Analytkonzentration im Körpergewebe eines Patienten. Indem bei einer erfindungsgemäß ausgebildeten Arbeitselektrode die Enzymschicht in Form von einzelnen Feldern beispielsweise über eine Distanz von mindestens drei Millimetern, bevorzugt mindestens fünf Millimetern, verteilt ist, wird erreicht, dass die Analytkonzentration in einem entsprechend großen Volumen gemessen wird. Störende Einflüsse, die nur ein kleines Volumenelement mit einem Durchmesser von etwa 0,1 mm betreffen, fallen bei einem erfindungsgemäßen Elektrodensystem deshalb nicht ins Gewicht. Überraschender Weise scheint ein beträchtlicher Teil der bei in-vivo Messungen mit bekannten Sensoren beobachteten Probleme einfach darauf zu beruhen, dass wegen der geringen Größe der Enzymschicht die Analytkonzentration in einem so kleinen Volumenelement gemessen wurde, dass dieses wegen transienter lokaler Effekte oft nicht für den übrigen Körper des Patienten repräsentativ ist.

Eine Ausdehnung der Enzymschicht über eine derartig große Strecke von einigen Millimetern und mehr macht eine Biegsamkeit der Arbeitselektrode erforderlich, damit sich deren Form bei Körperbewegungen anpassen kann. Die verhältnismäßig spröden Materialeigenschaften bekannter leitfähiger Enzymschichten scheinen eine biegsame Arbeitselektrode jedoch unmöglich zu machen. Durch die erfindungsgemäße Maßnahme, die Enzymschicht in Form von mehreren Feldern auszubilden, die auf dem Leiter der Arbeitselektrode in einem Abstand voneinander angeordnet sind, lässt sich aber trotz der spröden Eigenschaften des Materials der Enzymschicht erreichen, dass die Arbeitselektrode gebogen werden kann, ohne dass die Enzymschicht abplatzt.

Als Ursache für fehlerhafte Messungen mit herkömmlichen Arbeitselektroden, deren wirksame Fläche, also die Enzymschicht, sich über weniger als 0,2 Quadratmillimeter erstreckt, wird vermutet, dass durch Bewegungen eines Patienten vorübergehend ein Flüssigkeitsaustausch in einem kleinen Volumenelement verhindert werden kann, beispielsweise indem Zellen gegen die Arbeitselektrode gedrückt und interstitielle Flüssigkeit verdrängt wird oder indem Kapillargefäße komprimiert und dadurch verschlossen werden. Vermutlich kann es auf diese Weise dazu kommen, dass die Analytkonzentration in dem betreffenden Volumenelement in der unmittelbaren Umgebung der Arbeitselektrode nicht für den übrigen Körper des Patienten repräsentativ ist. Diese Volumenelemente, in denen ein Flüssigkeitsaustausch vorübergehend unterbunden ist, scheinen jedoch sehr klein zu sein und in der Regel nur einen Durchmesser von weniger als 1 mm zu haben. Wahrscheinlich bewirkt die elastische und weiche Beschaffenheit von Körpergewebe, dass Kräfte über sehr kurze Distanzen relaxieren können und der Austausch von Körperflüssigkeit deshalb nur in so kleinen Volumenelementen beeinträchtigt wird. Indem bei einem erfindungsgemäßen Elektrodensystem zumindest einige der die Enzymschicht bildenden Felder über eine erhebliche Distanz verteilt sind, beispielsweise voneinander mindestens 5 mm, vorzugsweise mindestens 1 cm, voneinander entfernt sind, wird deshalb erreicht, dass selbst im ungünstigsten Fall nur ein kleiner Teil der Arbeitselektrode, der in der Regel vernachlässigbar ist, beeinträchtigt ist.

Die Entfernung zwischen zwei Feldern der Enzymschicht einer erfindungsgemäßen Arbeitselektrode ist hierbei vom Rand des einen Feldes bis zu dem ihm zugewandten Rand des anderen Feldes zu messen.

Bevorzugt ist dabei, dass zwischen benachbarten Feldern ein Abstand von mindestens 0,3 Millimeter, insbesondere von mindestens 0,5 Millimeter ist. Die einzelnen Felder erstrecken sich bevorzugt in zwei zu einander senkrechten Richtungen jeweils weniger als zwei Millimeter, vorzugsweise weniger als einen Millimeter, insbesondere weniger als 0,6 Millimeter, weit. Die Felder können beispielsweise Kreise mit einem Durchmesser von weniger als einem Millimeter oder Rechtecke mit einer Kantenlänge von weniger als einem Millimeter sein. Bevorzugt sind die Felder auf dem Leiter der Arbeitselektrode in einer Reihe angeordnet. Möglich ist es aber beispielsweise auch die Felder in Zeilen und Spalten auf einem kreisförmigen oder rechteckigen Leiter anzuordnen. Die Anzahl der Felder kann nahezu beliebig gewählt werden. Bevorzugt weist die Arbeitselektrode mindestens 5 Felder auf.

Die Arbeitselektrode eines erfindungsgemäßen Elektrodensystems ist mit einer Diffusionsbremse versehen, welche die Diffusion des Analyten aus das Elektrodensystem umgebender Körperflüssigkeit zu in der Enzymschicht immobilisierten Enzymmolekülen verlangsamt. Die Diffusionsbremse kann beispielsweise eine die Enzymschicht bedeckende Deckschicht sein. Möglich ist es auch, dass in die Enzymschicht als Diffusionsbremse diffusionshemmende Partikel eingelagert sind. Beispielsweise können Poren der Enzymschicht mit einem Polymer gefüllt sein, durch welches Analytmoleküle nur langsam diffundieren können. Vorteilhaft lässt sich durch eine Diffusionsbremse der Verbrauch von Analytmolekülen an der Arbeitselektrode senken. Wird durch Bewegungen des Patienten vorübergehend der Austausch von Körperflüssigkeit in einer Umgebung eines Enzymschichtfeldes der Arbeitselektrode gestört, trägt eine geringere Umwandlungsrate von Analytmolekülen dazu bei, die Auswirkungen einer solchen Störung zu verringern. Je geringer nämlich der Analytverbrauch ist, desto länger dauert es, bis Verarmungseffekte auftreten, also die Analytkonzentration in dem betreffenden Bereich als Folge der vorgenommenen Messungen absinkt.

Eine vorteilhafte Weiterbildung der Erfindung sieht vor, dass die Arbeitselektrode einen Abstandshalter aufweist, der von dem Leiter aus gesehen über der Enzymschicht angeordnet ist und einen Mindestabstand zwischen der Enzymschicht und Zellen umgebenden Körpergewebes bewirkt. Der Abstandshalter bildet ein Reservoir für Analytmoleküle. Auf diese Weise lassen sich die Auswirkungen einer vorübergehenden Störung des Flüssigkeitsaustausches in der Umgebung der Arbeitselektrode weiter reduzieren. Der Abstandshalter kann beispielsweise eine Schicht aus einem biokompatiblen Polymer sein, das eine Permeation des Analyten ermöglicht. Mittels eines Abstandshalters lässt sich ein Puffervolumen für den Analyten schaffen, aus dem die Enzymfelder der Arbeitselektrode versorgt werden können. Vorteilhaft lässt sich auf diese Weise erreichen, dass selbst bei einer erheblichen Störung des Flüssigkeitsaustausches in Umgebung eines Enzymschichtfeldes über einen Zeitraum von einer halben Stunde keine merkliche Beeinträchtigung des Messsignals auftritt.

Der Abstandshalter kann beispielsweise auch als eine poröse Membran, beispielsweise eine Dialysemembran, oder ein Netz ausgebildet sein. Der Abstandshalter ist vorzugsweise drei Mikrometer bis 30 Mikrometer dick. Der Abstandshalter kann auf einer diffusionshemmenden Deckschicht angeordnet sein. Möglich ist es aber auch, den Abstandshalter unmittelbar auf der Enzymschicht anzuordnen. Der Abstandshalter kann dabei auch als Diffusionsbremse wirken und die Diffusion von Analytmolekülen zu der Enzymschicht verlangsamen.

Bevorzugt bedeckt der Abstandhalter als durchgehende Schicht die Arbeitselektrode und die Gegenelektrode sowie - falls vorhanden - auch die Referenzelektrode. Bevorzugt bedeckt der Abstandhalter die gesamte implantierte Fläche des Substrats. Durch eine Biokompatibilität des Abstandhalters lässt sich so die Gewebereaktion auf das Implantat verringern. Unabhängig davon ist ferner bevorzugt, dass der Abstandhalter auf einer diffusionshemmenden Deckschicht angeordnet ist und stärker hydrophil als die Deckschicht ist.

Der elektrische Leiter der Arbeitselektrode ist ebenso wie der elektrische Leiter der Gegenelektrode eines erfindungsgemäßen Elektrodensystems bevorzugt als eine Leiterbahn auf einem Substrat ausgebildet, beispielsweise eine Leiterbahn aus Metall oder Graphit auf einem Kunststoffplättchen. Möglich ist es jedoch auch, die Leiter als Drähte auszubilden. Die Enzymschichtfelder können auf einem als Draht ausgebildeten Leiter beispielsweise als ringförmige Segmente angeordnet werden. Die vorstehend erläuterte Angabe, dass sich die einzelnen Felder bevorzugt in zwei zu einander senkrechten Richtungen jeweils weniger als zwei Millimeter, vorzugsweise weniger als einen Millimeter, insbesondere weniger als 0,6 Millimeter, weit erstrecken, ist im Fall von ringförmigen Segmenten auf einem Draht dahingehend zu verstehen, dass die Breite des Rings und sein Durchmesser die beiden zueinander senkrechten Richtungen darstellen.

Sowohl die Verwendung von metallischen Drähten als auch von Leiterbahnen auf einem Substrat ermöglicht die Ausbildung eines flexiblen Sensors, der sich im Körper eines Patienten ohne zu brechen um 90 Grad und mehr biegen lässt.

Üblicherweise erstrecken sich Leiterbahnen nur auf einer einzigen Seite eines Substrates. Prinzipiell ist es jedoch auch möglich, dass sich ein einziger Leiter auf gegenüberliegenden Seiten eines Substrats erstreckt, beispielsweise durch eine Bohrung hindurch oder um eine seitliche Kante herum.

Eine weitere vorteilhafte Weiterbildung der Erfindung sieht vor, dass das Enzym in der Enzymschicht mit einem katalytischen Redoxmediator zusammenwirkt, der eine Sauerstoffabhängigkeit der katalytischen Umsetzung des Analyten reduziert oder verhindert. Derartige katalytische Redoxmediatoren werden manchmal auch als E-lektrokatalysatoren bezeichnet, da sie die Übertragung von Elektronen auf leitfähige Bestandteile der Arbeitselektrode, beispielsweise Graphitpartikel in der Enzymschicht, begünstigen. Als katalytische Redoxmediatoren können beispielsweise Mangandioxid in Form von Braunstein oder andere Metalloxide verwendet werden, welche Wasserstoffperoxid katalytisch oxidieren und dabei Elektronen auf leitfähige Bestandteile der Arbeitselektrode übertragen. Durch einen katalytischen Redoxmediator in Form eines Metalloxids kann zudem vorteilhaft das Potential der Arbeitselektrode um mehr als 100 Millivolt gesenkt werden, so dass sich der Einfluss interferierender Substanzen, beispielsweise Ascorbat oder Harnsäure, auf das Messsignal erheblich reduziert. Bei Enzymen, die Analytmoleküle oxidieren und dabei Wasserstoffperoxid erzeugen, lässt sich durch Verwendung eines solchen katalytischen Redoxmeditors einer Verarmung von Sauerstoff in der Umgebung der Arbeitselektrode entgegenwirken und folglich erreichen, dass die Umsetzgeschwindigkeit in einem weiten Konzentrationsbereich nur von der Analytkonzentration aber nicht von der Sauerstoffkonzentration abhängt.

Als katalytische Redoxmediatoren, die Wasserstoffperoxid abbauen, sind auch metallorganische Verbindungen, beispielsweise Cobalt-Phtalocyanin geeignet. Der katalytische Redoxmediator kann kovalent an die Enzymmoleküle gebunden sein oder beispielsweise in Form von separaten Partikeln in die Enzymschicht eingebettet sein.

Möglich ist es auch, dass der katalytische Redoxmediator einen direkten Elektrontransfer bewirkt. Mit einem kovalent an das Enzym gebundenen katalytischen Redoxmeditor lässt sich auf diese Weise eine Oxidation von Analytmolekülen und ein Elektronübertrag auf die Arbeitselektrode ohne den Zwischenschritt einer Erzeugung von Wasserstoffperoxid bewirken. Bei einem direkten Elektrontransfer wird ein Elektron aus einer prosthischen Gruppe des Enzyms direkt auf den katalytischen Redoxmediator und von dort auf einen leitfähigen Bestandteil der Arbeitselektrode, beispielsweise Graphitpartikel in der Enzymschicht, übertragen.

Ein direkter Elektrontransfer lässt sich beispielsweise mit Enzymen, wie Dehydrogenasen, bewirken, die Pyrrolochinolinchinon (PQQ) als prosthische Gruppe haben. Im Fall von Glukosedehydrogenase (GlucDH) aus Acinetobacter calcoaceticus wird PQQ bei Oxidation der Glukose durch das Enzym in einen reduzierten Zustand versetzt. Diese prosthische Gruppe kann kovalent über Gold-Nanopartikel direkt an einen Leiter gebunden werden, um so einen direkten Elektronübergang vom reduzierten PQQ auf den Leiter zu ermöglichen. Sauerstoff reagiert offensichtlich nicht mit reduziertem PQQ, tritt also nicht mit dem Elektronübertrag in Konkurrenz. Ein andere Weg, die Elektronen vom reduzierten PQQ zu übertragen, beruht auf der Erkenntnis, dass PQQ selbst in mehreren Oxidationsstufen

$$PQQ + e^- + H^+ \leftrightarrow PQQH^{\cdot}$$

$$PQQH + e^- + H^+ \leftrightarrow PQQH_2$$

vorliegen kann, und daher als katalytischer Redoxmediator einsetzbar ist. Demzufolge können zusätzliche PQQ-Moleküle kovalent auf dem Enzym GlucDH gebunden werden, die zur Aufnahme von Elektronen aus einer katalytisch aktiven Tasche des Proteins, also von der dort befindlichen prosthischen Gruppe PQQ, dienen.

Eine weitere Möglichkeit beruht auf einer Variante der GlucDH (aus Burkholderia cepacia), die als prostethische Gruppe Flavin-Adenin-Dinucleotid (FAD) hat, und in einer anderen Untereinheit das Proteins Cytochrom-C aufweist, welches Elektronen von $FADH_2$ übertragen kann.

Ein erfindungsgemäßes Elektrodensystem kann zusätzlich eine Referenzelektrode aufweisen. Die Referenzelektrode kann ein Bezugspotential für die Arbeitselektrode liefern, das beispielsweise durch das Redoxsystem Silber/Silberchlorid definiert wird. Ferner kann ein erfindungsgemäßes Elektrodensystem weitere Elektroden, beispielsweise zusätzliche Arbeitselektroden aufweisen, insbesondere Arbeitselektroden mit unterschiedlicher Messsensitivität wie es in der US 2007/0151868 A1 beschrieben ist.

Ein erfindungsgemäßes Elektrodensystem bildet zusammen mit einem an das Elektrodensystem angeschlossen Potentiostaten und einem Verstärker zur Verstärkung von Messsignalen einen Sensor. Bevorzugt sind der Verstärker und der Potentiostat auf einer Leiterplatte angeordnet, welche die Leiter der Gegenelektrode und der Arbeitselektrode trägt. Beispielsweise können die Elektroden auf einem Substrat in Form eines Kunststoffplättchens angeordnet sein, dass mit einem Ende auf der Leiterplatte befestigt ist. Möglich ist es auch, die Leiterplatte in das Substrat, auf dem die Elektroden angeordnet sind, zu integrieren. Beispielsweise können der Potentiostat und der Vorverstärker auf einem biegsamen Kunststoffplättchen angeordnet sein, dass zugleich eine Leiterplatte und ein Substrat für Leiterbahnen des Elektrodensystems bildet.

Weitere Einzelheiten und Vorteile der Erfindung werden an einem Ausführungsbeispiel unter Bezugnahme auf die beigefügten Zeichnungen erläutert. Es zeigen:

Figur 1: ein Ausführungsbeispiel eines erfindungsgemäßen Elektrodensystems;

Figur 2: eine Detailansicht zu Figur 1;

Figur 3: eine weitere Detailansicht zu Figur 1;

Figur 4: einen Schnitt entlang der Schnittlinie CC der Figur 2;

Figur 5: in-vitro Funktionskurven des in Figur 1 gezeigten Elektrodensystems mit unterschiedlicher Deckschicht;

Figur 6: Beispiele von in-vivo Messungen erfindungsgemäßer Elektrodensyste-men; und

Figur 7 Vergleichsmessungen in-vitro eines Elektrodensystems bei verschiede-nen Sauerstoffkonzentrationen.

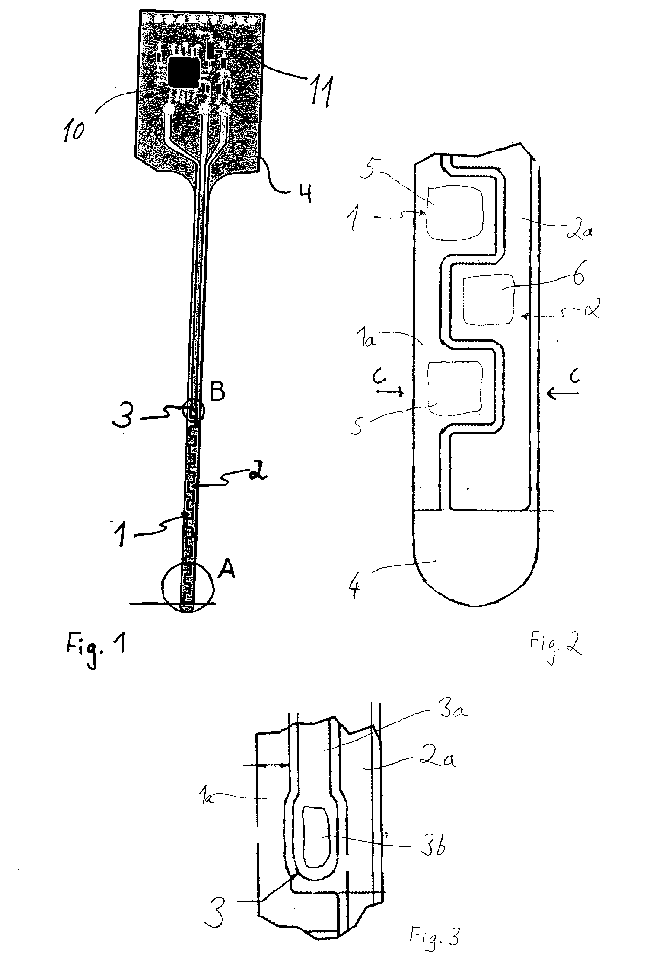

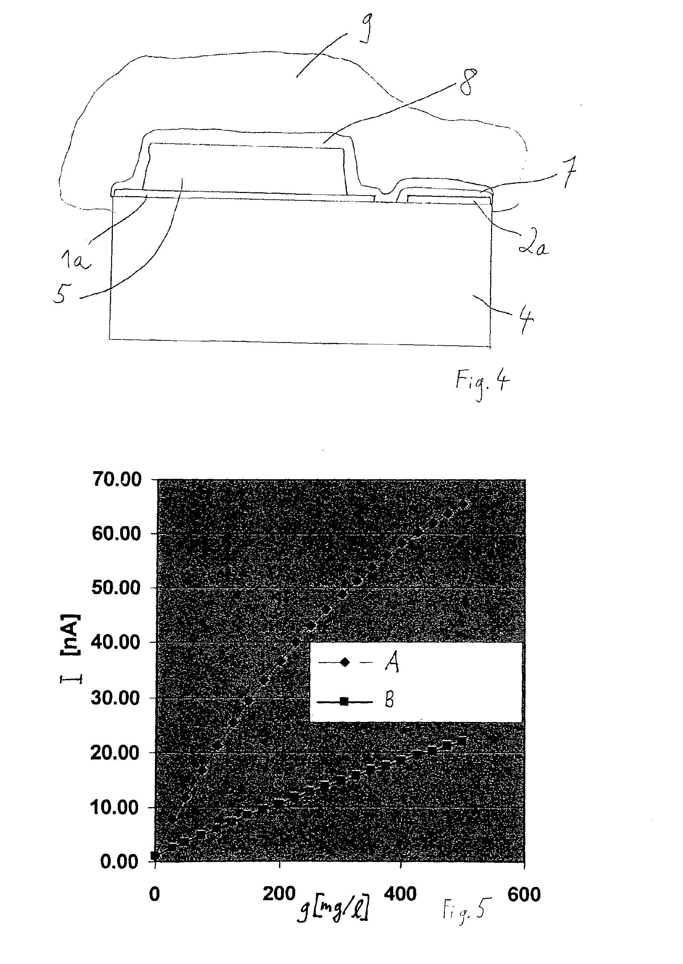

Figur 1 zeigt ein Ausführungsbeispiel eines Elektrodensystems zur Insertion in Körpergewebe eines Menschen oder Tieres, beispielsweise Cutis oder Unterhautfettgewebe. Eine Vergrößerung des Bildausschnitts A ist in Figur 2, eine Vergrößerung des Bildausschnitts B in Figur 3 dargestellt. Figur 4 zeigt eine dazugehörende Schnittansicht entlang der Schnittlinie CC der Figur 2.

Das dargestellte Elektrodensystem hat eine Arbeitselektrode 1, eine Gegenelektrode 2 und eine Referenzelektrode 3. Elektrische Leiter der Elektroden 1 a, 2a, 3a sind als metallische Leiterbahnen, bevorzugt aus Palladium oder Gold, auf einem Substrat 4 angeordnet. Bei dem dargestellten Ausführungsbeispiel ist das Substrat 4 ein biegsames Kunststoffplättchen, beispielsweise aus Polyester. Das Substrat 4 hat eine Stärke von weniger als 0,5 mm, beispielsweise 100 bis 300 Mikrometern, und lässt sich deshalb leicht biegen, so dass es sich nach Insertion an Bewegungen umgebenden Körpergewebes anpassen kann. Das Substrat 4 hat einen schmalen Schaft zur Insertion im Körpergewebe eines Patienten und einen breiten Kopf zum Anschließen an eine außerhalb des Körpers angeordnete Elektronik. Der Schaft des Substrates 4 hat eine Länge von vorzugsweise mindestens 1 cm, insbesondere 2 cm bis 5 cm.

Die Arbeitselektrode 1 trägt eine Enzymschicht 5, die immobilisierte Enzymmoleküle zur katalytischen Umwandlung des Analyten enthält. Die Enzymschicht 5 kann beispielsweise als eine aushärtende Paste aus Kohlenstoffpartikeln, einem polymeren Bindemittel und Enzymmolekülen aufgetragen werden. Einzelheiten zur Herstellung einer solchen Enzymschicht 5 sind beispielsweise in der WO 2007/147475 offenbart, auf die diesbezüglich verwiesen wird. Bei dem zu messenden Analyten kann es sich beispielsweise um Glukose, Laktat oder andere medizinisch bedeutsame Moleküle handeln. Als Enzym wird in der Regel eine Oxidase verwendet, also beispielsweise eine Glucoseoxidase oder Laktatoxidase, oder eine Dehydrogenase, also beispielsweise eine Glucosedehydrogenase.

In dem dargestellten Ausführungsbeispiel ist die Enzymschicht 5 auf dem Leiter 1 a der Arbeitselektrode 1 aber nicht durchgehend aufgetragen, sondern in Form von einzelnen Feldern ausgebildet, die in einem Abstand voneinander angeordnet sind. Obwohl die Enzymschicht 5 spröde ist, wird auf diese Weise erreicht, dass sich das Elektrodensystem biegen lässt, ohne dass es zu einem Abplatzen der Enzymschicht 5 kommt. Das dargestellte Elektrodensystem lässt sich deshalb ohne zu brechen um über 90° biegen, so dass es sich nach Insertion an Körperbewegungen anpassen kann.

Die einzelnen Felder der Enzymschicht 5 sind bei dem dargestellten Ausführungsbeispiel in einer Reihe angeordnet, wobei zwischen dem ersten und dem letzten Feld dieser Reihe ein Abstand von mehr als 1 cm ist. Zwischen benachbarten Feldern ist jeweils ein Abstand von mindestens 0,3 mm, insbesondere mehr als 0,5 mm, wobei der Abstand von Feldrand zu Feldrand zu messen ist. Die einzelnen Felder erstrecken sich in zwei zueinander senkrechten Richtungen jeweils 0,2 mm bis 0,4 mm weit. Die Form der Felder kann beispielsweise kreisförmig oder quadratisch sein. Die Gesamtfläche aller Felder zusammen kann praktisch beliebig gewählt werden. Im Allgemeinen ist eine Gesamtfläche von weniger als einem Quadratmillimeter ausreichend. Bei dem dargestellten Ausführungsbeispiel beträgt die Gesamtfläche etwa 0,4 bis 0,6 Quadratmillimeter.

Der Leiter 1a der Arbeitselektrode 1 weist zwischen den Enzymschichtfeldern Schmalstellen auf, die insbesondere in Figur 2 zu sehen sind. Der Leiter 2a der Gegenelektrode 2 weist eine dem Verlauf des Leiters 1a der Arbeitselektrode 1 folgende Kontur auf. Auf diese Weise ergibt sich eine interkalierende oder verzahnte Anordnung von Arbeitselektrode 1 und Gegenelektrode 2 mit vorteilhaft kurzen Strompfaden und geringer Stromdichte. Der Leiter 1a der Arbeitselektrode 1 ist bei dem dargestellten Ausführungsbeispiel relativ schmal ausgebildet und hat eine Breite von weniger als 1 mm. Bei dem dargestellten Ausführungsbeispiel hat der Leiter 1a an seinen breiten Stellen, die mit Feldern der Enzymschicht 5, belegt sind, eine Breite von weniger als 0,6 mm, nämlich etwa 0,3 mm bis 0,5 mm. An den dazwischen liegenden schmalen Stellen haben die Leiter 1a und 2a eine Breite von weniger als 0,3 mm, nämlich 0,05 mm bis 0,2 mm.

Zur Erhöhung ihrer wirksamen Oberfläche kann die Gegenelektrode 2 mit einer porösen, elektrisch leitfähigen Schicht 6 versehen sein, die in Form von einzelnen Feldern auf dem Leiter 2a der Gegenelektrode 2 liegen. Diese Schicht 6 kann ebenso wie die Enzymschicht 5 der Arbeitselektrode 1 als eine aushärtende Paste aus Kohlenstoffpartikeln und einem polymeren Bindemittel aufgetragen werden. Bevorzugt haben die Felder der Schicht 6 dieselben Abmessungen wie die Felder der Enzymschicht 5, erforderlich ist dies jedoch nicht. Auf Maßnahmen zur Erhöhung der Oberflache der Gegenelektrode kann jedoch auch verzichtet werden und die Gegenelektrode 2 beispielsweise auch als eine geradlinige Leiterbahn ohne irgendwelche Beläge ausgebildet werden.

Die Referenzelektrode 3 ist zwischen dem Leiter 1a der Arbeitselektrode 1 und dem Leiter 2a der Gegenelektrode 2 angeordnet. Die in Figur 3 gezeigte Referenzelektrode besteht aus einem Leiter 3a, auf dem ein Feld 3b aus leitfähiger Silber/Silberchloridpaste angeordnet ist.

Figur 4 zeigt eine schematische Schnittansicht entlang der Schnittlinie CC der Figur 2. Die Schnittlinie CC verläuft durch eines der Enzymschichtfelder 5 der Arbeitselektrode 1 und zwischen den Feldern der leitfähigen Schicht 6 der Gegenelektrode 2. Zwischen den Feldern der Enzymschicht 5 kann der Leiter 1a der Arbeitselektrode 1 ebenso wie der Leiter 2a der Gegenelektrode 2 zwischen den Feldern der leitfähigen Schicht 6 mit einer Isolationsschicht 7 bedeckt sein, um störende Reaktionen zu vermeiden, die andernfalls durch das Metall der Leiterbahnen 1a, 2a katalysiert werden könnten.

Die Enzymschicht 5 ist von einer Deckschicht 8 bedeckt, welche dem zu messenden Analyten einen Diffusionswiderstand entgegensetzt und deshalb als Diffusionsbremse wirkt. Die Deckschicht 8 kann beispielsweise aus Polyurethan, einem Acrylat, insbesondere einem Copolymer aus Methylmethacrylat und Hydroxyethylmethacrylat, oder einem anderen schwach quellenden Polymer sein. Eine günstige Stärke der Deckschicht 8 sind beispielsweise 3 bis 30 Mikrometer. Die Deckschicht 8 bewirkt wegen ihres Diffusionswiderstandes, dass pro Zeiteinheit weniger Analytmoleküle zu der Enzymschicht 5 gelangen. Durch die Deckschicht 8 wird deshalb die Rate, mit der Analytmoleküle umgesetzt werden, reduziert und somit einer Verarmung der Analytkonzentration entgegengewirkt.

Die Deckschicht 8 erstreckt sich kontinuierlich im wesentlichen über die gesamte Fläche des Leiters 1a der Arbeitselektrode 1. Auf der Deckschicht 8 ist als Abstandshalter 9 eine biokompatible Membran angeordnet, die einen Mindestabstand zwischen der Enzymschicht 5 und Zellen umgebenden Körpergewebes bewirkt. Vorteilhaft wird auf diese Weise ein Reservoir für Analytmoleküle geschaffen, aus dem bei einer vorübergehenden Störung des Flüssigkeitsaustausches in der Umgebung eines Enzymschichtfeldes 5 Analytmoleküle zu dem betreffenden Enzymschichtfeld 5 gelangen können. Wird vorübergehend der Austausch von Körperflüssigkeit in der Umgebung des Elektrodensystems eingeschränkt oder sogar unterbunden, diffundieren die in dem Abstandshalter 9 gespeicherten Analytmoleküle nämlich weiterhin zu der Enzymschicht 5 der Arbeitselektrode 1, in der sie umgewandelt werden. Der Abstandshalter 9 bewirkt deshalb, dass eine merkliche Verarmung der Analytkonzentration und eine entsprechende Verfälschung der Messergebnisse erst nach einer wesentlich größeren Zeitspanne auftritt. Bei dem dargestellten Ausführungsbeispiel bedeckt die den Abstandhalter 9 bildende Membran auch die Gegenelektrode 2 sowie die Referenzelektrode 3.

Die Abstandhaltermembran 9 kann beispielsweise eine Dialysemembran sein. Unter einer Dialysemembran wird in diesem Zusammenhang eine Membran verstanden, die für Moleküle oberhalb einer Maximalgröße undurchlässig ist. Die Dialysemembran kann in einem separaten Herstellungsprozess vorgefertigt und als vollständige, bestehende Struktur bei der Fertigung des Elektrodensystems aufgebracht werden. Die Maximalgröße der Moleküle, für welche die Dialysemembran durchlässig ist, wird so gewählt, dass Analytmoleküle hindurch treten können, größere Moleküle jedoch abgehalten werden.

Alternativ zu einer Dialysemembran kann als Abstandhaltermembran 9 auch ein Überzug aus einem für den Analyten und Wasser sehr permeablen Polymer über das Elektrodensystem gelegt werden, beispielsweise auf Basis von Polyurethan.

Die Enzymschicht 5 kann Metalloxidpartikel, bevorzugt Mangandioxidpartikel, als katalytischen Redoxmediator enthalten. Mangandioxid setzt katalytisch Wasserstoffperoxid um, das beispielsweise bei der enzymatischen Oxidation von Glukose und anderen Bioanalyten gebildet wird. Beim Abbau von Wasserstoffperoxid übertragen die Mangandioxidpartikel Elektronen auf leitfähige Bestandteile der Arbeitselektrode 1, beispielsweise auf Graphitpartikel in der Enzymschicht 5. Durch den katalytischen Abbau von Wasserstoffperoxid wird einem Absinken der Sauerstoffkonzentration in der Enzymschicht 5 entgegengewirkt. Vorteilhaft kann so erreicht werden, dass die Umsetzung des nachzuweisenden Analyten in der Enzymsicht 5 nicht durch die lokale Sauerstoffkonzentration begrenzt ist. Der Einsatz eines katalytischen Redoxmediators wirkt deshalb einer Verfälschung des Messsignals durch eine geringe Sauerstoffkonzentration entgegen. Ein weiterer Vorteil eines katalytischen Redoxmediators besteht darin, ein Entstehen zellschädigender Konzentrationen von Wasserstoffperoxid zu verhindern.

Figur 5 zeigt in-vitro gemessene Funktionskurven des beschriebenen Elektrodensystems mit unterschiedlichen Deckschichten 8. Als Funktionskurven sind die Stärke des gemessenen Stroms in nA als Funktion der Glukosekonzentration in mg/dl aufgetragen. Die obere Funktionskurve A wurde mit einem Elektrodensystem gemessen, dessen Deckmembran 8 aus hydrophilisiertem Polyurethan eine Stärke von fünf Mikrometern hat. Im Vergleich dazu ist als untere Funktionskurve B die Abhängigkeit des Stromes von der Glukosekonzentration für ein Elektrodensystem mit einer Deckmembran 8 gezeigt, die Analytmolekülen einen etwa doppelt so großen Diffusionswiderstand entgegensetzt, beispielsweise wegen einer entsprechend größeren Stärke oder einer geringeren Hydrophilisierung. Die Elektrodensysteme der in Figur 5 dargestellten Funktionskurven wurden mit einer Polarisationsspannung von 350 mV betrieben.

Die als Deckschichten verwendeten hydrophilisierten Polyurethane (HPU) können durch Polykondensation von 4,4'-Methylen-bis (cyclohexylisocyanat) mit Diol-Gemischen hergestellt werden. Die beiden Komponenten des Diol-Gemisches, die zur Einstellung des Hydrophilisierungsgrades des Polymers benutzt wurden, sind Polyethylenglykol (PEG, MW (Molecular Weight) = 1000 g/mol) und Polypropylenglykol (PPG, MW (Molecular Weight) = 1500 g/mol). Für die Funktionskurve A wurde die Deckschicht 8 aus HPU mit PEG zu PPG im Verhältnis 1 zu 3 erzeugt. Für die Funktionskurve B wurde die Deckschicht 8 aus HPU mit PEG:PPG entsprechend 1:7 erzeugt. In beiden Fällen ist die Deckschicht 8 ca. 5 $\mu$m dick.

Damit die Analytkonzentration in der Umgebung des Elektrodensystems durch die Messung möglichst wenig beeinflusst wird und deshalb auch bei einer vorübergehenden Störung des Austauschs von Körperflüssigkeit allenfalls geringfügig verfälscht wird, sind geringe Umsetzraten des Analyten und somit geringe Messströme vorteilhaft. Gute Ergebnisse lassen sich mit Elektrodensystemen erzielen, die mit einer Gesamtfläche der Enzymschicht von 1 mm$^2$ oder weniger bei einer Glukosekonzentration von 180 mg/dl einen Strom von weniger als 50 nA, insbesondere als 10 nA, liefern. Beispielsweise wurde mit dem Elektrodensystem der in Figur 5 dargestellten Funktionskurve B in der Cutis eines Schweins bei einer Glucosekonzentration von 180 mg/dl ein Strom von 3 nA gemessen. Derartig kleine Messsignale lassen sich schlecht über größere Entfernungen übertragen. Bevorzugt sind deshalb ein Potentiostat und ein Verstärker in unmittelbarer Nähe des Elektrodensystems angeordnet. Beispielsweise können ein Potentiostat 10 und ein Verstärker 11 auf einem Kopf des Substrats 4 angeordnet sein, wie dies in Figur 1 dargestellt ist. Möglich ist es auch, das Substrat 4 auf einer Leiterplatte zu befestigen, welche den Potentiostaten und den Verstärker trägt.

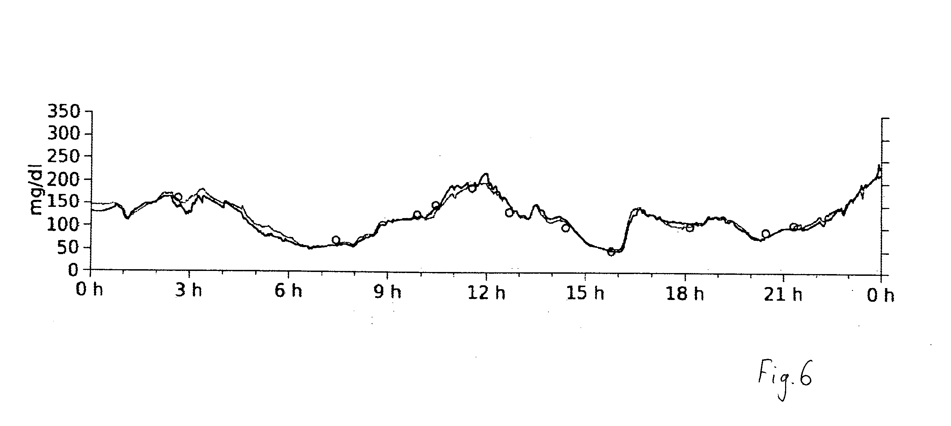

Figur 6 zeigt in-vivo Messungen, die mit den beiden Elektrodensystemen, deren Funktionskurven in Figur 5 gezeigt sind und die zusätzlich mit einem Abstandshalter versehen wurden, im Unterhautfettgewebe abdominal bei einem insulinpflichtigen Diabetiker gemessen wurden. Die beiden Elektrodensysteme wurden in einem Abstand von etwa 10 cm implantiert.

An dem typischen Verlauf Signale der beiden zugleich implantierten Sensoren erkennt man, dass bei Verwendung erfindungsgemäßer Elektrodensysteme eine hervorragende Übereinstimmung der Ergebnisse erzielt wird. Zwischen den beiden Insertionsstellen bestehen keine relevanten Abweichungen in der lokalen Glukosekonzentration. Weiterhin belegen die dargestellten Ergebnisse, dass auch keine transienten Abweichungen in der Glukosekonzentration zwischen Blut und Gewebe zu bestehen scheinen.

Die Stromwerte der beiden Sensoren wurden mit einer Samplingrate von einem Messwert pro Minute ohne Filterung in Glukosewerte umgerechnet. Die Umrechnung erfolgte mithilfe von Blutzuckerwerten, die an Körperflüssigkeitsproben ex-vivo bestimmt wurden.

Für die in Figur 6 gezeigten in-vivo Messungen wurde das Elektrodensystem nach Aufbringen der Deckschicht 8 aus hydrophilem Polyurethan in eine 12.5%ige ethanolische Lösung des Copolymers aus Butylmethacrylat (BMA) und 2-Methacryloyloxyethyl-phosphorylcholin (MPC) (Lipidure Cm5206, NOF Corp, Japan) getaucht und anschließend der so erzeugte 25 $\mu$m dicke Überzug 12 h getrocknet.

Die Stromdichte verändert sich durch diesen Abstandhalter 9 aus BMA-MPC praktisch nicht: ohne den Abstandhalter 9 erreicht das Elektrodensystem aus Figur 5, Funktionskurve A, ca. 40 nA/mm$^2$ bei 180 mg/dl, mit Abstandshalter aus BMA-MPC werden 38 nA/mm$^2$ erzielt. Beim Elektrodensystem der Funktionskurve B ist gar kein Unterschied in der Stromhöhe feststellbar: 10 nA/mm$^2$ bei 180 mg/dl mit und ohne Abstandshalter 9 aus BMA-MPC.

Der Abstandshalter unterdrückt in-vivo Bewegungseinflüsse auf den Sensor. So wird in diesem Ausführungsbeispiel der Amplituden-Anteil an der Fluktuation des Sensorsignals, welcher eindeutig auf Bewegungseffekt zurückgeführt werden kann, durch den Abstandshalter von 5 bis 25% der mittleren Signalhöhe auf 0.5 bis 5% der mittleren Signalhöhe zurückgeführt.

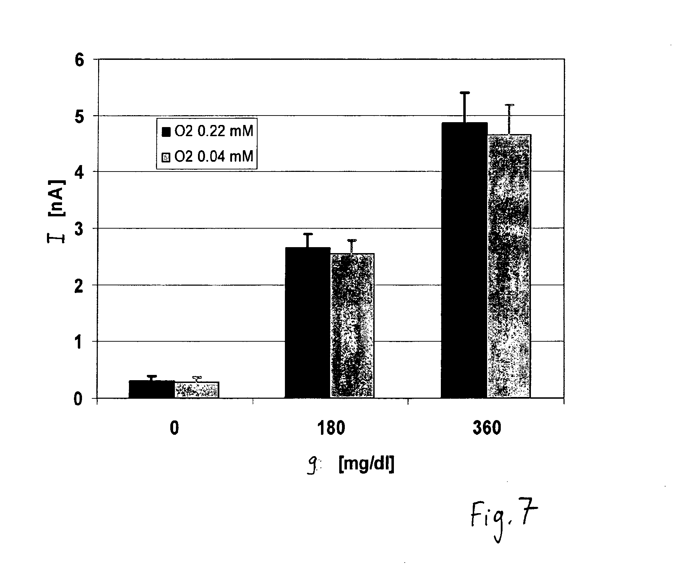

Figur 7 zeigt als Balkendiagramm die in-vitro gemessene Stromstärke I für drei verschiedene Glucosekonzentrationen g, nämlich 0 mg/dl, 180 mg/dl und 360 mg/dl bei jeweils zwei verschiedenen Sauerstoffkonzentrationen, nämlich 0,22 mmol/l (jeweils linker Balken) und 0.04mmol/l (jeweils rechter Balken der dargestellten Balkenpaare). Die Messungen wurden für das vorstehend beschriebene Elektrodensystem durchgeführt, wobei die Enzymschicht 5 so aufgebaut ist, dass ein direkter Elektrontransfer gewährleistet ist. Als Enzym wird GlucDH (EC 1.1.99.17) aus Acinetobacter calcoaceticus eingesetzt. In einem ersten Schritt werden zunächst zusätzliche PQQ-Moleküle als katalytischer Redoxmediator kovalent an GlucDH gebunden, beispielsweise durch Versetzen des PQQ-Säurechlorids mit dem Enzym. In einem zweiten Schritt wird eine Graphit-haltige Paste zur Verbesserung der Leitfähigkeit und der Porösität mit Carbon-Nanotubes (NanoLab, Newton, MA, USA; multiwall CNT, research grade) versetzt, dann mit der PQQ modifizierte GlucDH gemischt und die so erzeugte Arbeitselektrodenpaste auf die Leiterbahn 1 a in verteilter Anordnung aufgedruckt, danach bei 40°C für 4h im Vakuum ausgehärtet. Das Elektrodensystem wurde zuvor mit Isolationsschicht 7, einer Referenzelektrode 3 und einer Gegenelektrode 2 mit leitfähiger Schicht 6 versehen. Nicht immobilisiertes Enzym wird durch Spülen mit Phosphatpuffer entfernt.

Auf die so entstandene Enzymschicht 5 wird eine Deckschicht 8 aus hydrophilisiertem Polyurethan (HPU, Verhältnis Polyethylenglykol: Polypropylenglykol = 1:3) dreifach als 2.5%iger ethanolischer Lösung aufdispensiert und 24h bei Raumtemperatur getrocknet. Die Dicke der entstandenen Deckschicht beträgt 2 μm. Zur Messung der in-vitro Funktion werden die Elektrodensysteme in Glukosemesslösung bei verschiedenen Sauerstoffkonzentrationen mit 200 mV Polarisationsspannung betrieben. Für 4 Sensoren werden jeweils Mittelwert und Standardabweichung des Messstroms berechnet. Figur 7 zeigt diese Werte für eine normale Sauerstoffsättigung der Messlösung von ca. 0,22 mmol/l und eine deutlich reduzierte Sauerstoffkonzentration von 0.04mmol/l. Man beobachtet keinen relevanten oder signifikanten Einfluss der Sauerstoffkonzentration auf die in-vitro Funktion für das Elektrodensystem mit direktem Elektronübertrag.

**Bezugszahlen**

1    Arbeitselektrode
1 a  Elektrischer Leiter der Arbeitselektrode
2    Gegenelektrode
2a  Elektrischer Leiter der Gegenelektrode
3    Referenzelektrode
3a  Elektrischer Leiter der Referenzelektrode

3b  Silber/Silberchlorid Schicht
4    Substrat
5    Enzymschicht
6    Leitfähige Schicht
7    Isolationsschicht
8    Deckschicht
9    Abstandshalter
10  Potentiostat
11  Verstärker

**Patentansprüche**

1. Elektrodensystem zur in-vivo Messung der Konzentration eines Analyten, umfassend eine Gegenelektrode (2) mit einem elektrischen Leiter (2a), eine Arbeitselektrode (1) mit einem elektrischen Leiter (1a) auf dem eine Enzymschicht (5) angeordnet ist, die immobilisierte Enzymmoleküle zur katalytischen Umwandlung des Analyten enthält, und einer Diffusionsbremse (8), welche die Diffusion des Analyten aus das Elektrodensystem umgebender Körperflüssigkeit zu Enzymmolekülen verlangsamt, **dadurch gekennzeichnet, dass** die Enzymschicht (5) in Form von mehreren Feldern ausgebildet ist, die auf dem Leiter (1a) der Arbeitselektrode (1) in einem Abstand von einander angeordnet sind.

2. Elektrodensystem nach Anspruch 1, **dadurch gekennzeichnet, dass** mindestens zwei der Felder der Enzymschicht (5) von einander mindestens 3mm, vorzugsweise mindestens 5 mm, entfernt sind.

3. Elektrodensystem nach einem der vorstehenden Ansprüche, **dadurch gekennzeichnet, dass** zwischen benachbarten Feldern der Enzymschicht (5) ein Abstand von mindestens 0,3 mm ist.

4. Elektrodensystem nach einem der vorstehenden Ansprüche, **dadurch gekennzeichnet, dass** sich die Felder der Enzymschicht (5) in zwei zueinander senkrechten Richtungen jeweils weniger als 2 mm weit erstrecken.

5. Elektrodensystem nach einem der vorstehenden Ansprüche, **dadurch gekennzeichnet, dass** die Diffusionsbremse (8) als eine die Enzymschicht (5) bedeckende Schicht ausgebildet ist

6. Elektrodensystem nach einem der vorstehenden Ansprüche, **dadurch gekennzeichnet, dass** das Enzym mit einem katalytischen Redoxmediator zusammenwirkt, der eine Sauerstoffabhängigkeit der katalytischen Umsetzung des Analyten reduziert oder verhindert.

7. Elektrodensystem nach Anspruch 6, **dadurch gekennzeichnet, dass** der katalytische Redoxmediator Wasserstoffperoxid umsetzt.

8. Elektrodensystem nach Anspruch 6 oder 7, **dadurch gekennzeichnet, dass** der katalytische Redoxmediator einen direkten Elektronentransfer bewirkt.

9. Elektrodensystem nach einem der vorstehenden Ansprüche, **dadurch gekennzeichnet, dass** sich das Elektrodensystem ohne zu brechen um 90° biegen lässt.

10. Elektrodensystem nach einem der vorstehenden Ansprüche, **dadurch gekennzeichnet, dass** die Arbeitselektrode (1) einen Abstandshalter (9) aufweist, der von dem Leiter (1a) aus gesehen über der Enzymschicht (5) angeordnet ist.

11. Elektrodensystem nach einem der vorstehenden Ansprüche, **dadurch gekennzeichnet, dass** der Abstandshalter (9) als durchgehende Schicht die Arbeitselektrode (1) und die Gegenelektrode (2) bedeckt.

12. Elektrodensystem nach einem der vorstehenden Ansprüche, **dadurch gekennzeichnet, dass** der elektrische Leiter (1 a) der Arbeitselektrode (1) und der elektrische Leiter (2a) der Gegenelektrode (2) auf einem Substrat (4) angeordnet sind.

13. Elektrodensystem nach einem der vorstehenden Ansprüche, **dadurch gekennzeichnet, dass** der Leiter (1a) der Arbeitselektrode (1) zwischen den Enzymschichtfeldern (5) Schmalstellen aufweist und der Leiter (2a) der Gegenelektrode (2) eine dem Verlauf des Leiters (1a) der Arbeitselektrode (1) folgende Kontur aufweist.

14. Sensor mit einem Elektrodensystem nach einem der vorstehenden Ansprüche, einem an das Elektrodensystem angeschlossenen Potentiostaten und einem Verstärker zur Verstärkung von Messsignalen des Elektrodensystems.

15. Sensor nach Anspruch 14, **dadurch gekennzeichnet, dass** die Elektroden (1, 2, 3) des Elektrodensystems auf einem Substrat (4) angeordnet sind, das den Potentiostaten (10) trägt oder auf einer Leiterplatte befestigt ist, die den Potentiostaten (10) trägt.

Fig. 1

Fig. 2

Fig. 3

Fig. 4

Fig. 5

Fig.6

EP 2 163 190 A1

Fig. 7

Europäisches
Patentamt

European
Patent Office

Office européen
des brevets

**EUROPÄISCHER RECHERCHENBERICHT**

**Nummer der Anmeldung**

EP 08 01 5982

## EINSCHLÄGIGE DOKUMENTE

| Kategorie | Kennzeichnung des Dokuments mit Angabe, soweit erforderlich, der maßgeblichen Teile | Betrifft Anspruch | KLASSIFIKATION DER ANMELDUNG (IPC) |
|---|---|---|---|
| Y | US 2007/208243 A1 (GABRIEL JEAN-CHRISTOPHE P [US] ET AL) 6. September 2007 (2007-09-06) * Absätze [0029], [0050] - [0053], [0061], [0066], [0088] - [0090]; Abbildungen 2A,7 * | 1-8, 10-15 | INV. A61B5/00 |
| Y | EP 1 785 085 A (ROCHE DIAGNOSTICS GMBH [DE]; HOFFMANN LA ROCHE LTD F [CH]) 16. Mai 2007 (2007-05-16) * Absätze [0030], [0031], [0037]; Abbildung 4 * | 1-8, 10-12, 14,15 | |
| A | EP 1 733 676 A (HOFFMANN LA ROCHE [CH]; ROCHE DIAGNOSTICS GMBH [DE]) 20. Dezember 2006 (2006-12-20) | | |
| Y | * Seite 3, Absatz 29 - Seite 4, Absatz 30 * | 13 | |
| D,A | US 4 655 880 A (LIU CHUNG-CHIUN [US]) 7. April 1987 (1987-04-07) * Spalte 11, letzter Absatz; Abbildungen 11,12 * | 1 | **RECHERCHIERTE SACHGEBIETE (IPC)** A61B C12M |
| D,A | WO 2007/147475 A (ROCHE DIAGNOSTICS GMBH [DE]; HOFFMANN LA ROCHE [CH]; STAIB ARNULF [DE]) 27. Dezember 2007 (2007-12-27) * Seite 8 - Seite 9; Abbildung 1 * | 5,10,11 | |
| D,A | US 2005/059871 A1 (GOUGH DAVID A [US] ET AL) 17. März 2005 (2005-03-17) * Absatz [0009] - Absatz [0011]; Abbildung 3 * | 1 | |

Der vorliegende Recherchenbericht wurde für alle Patentansprüche erstellt

| Recherchenort | Abschlußdatum der Recherche | Prüfer |
|---|---|---|
| München | 22. Dezember 2008 | Schindler, Martin |

KATEGORIE DER GENANNTEN DOKUMENTE

X : von besonderer Bedeutung allein betrachtet
Y : von besonderer Bedeutung in Verbindung mit einer anderen Veröffentlichung derselben Kategorie
A : technologischer Hintergrund
O : nichtschriftliche Offenbarung
P : Zwischenliteratur

T : der Erfindung zugrunde liegende Theorien oder Grundsätze
E : älteres Patentdokument, das jedoch erst am oder nach dem Anmeldedatum veröffentlicht worden ist
D : in der Anmeldung angeführtes Dokument
L : aus anderen Gründen angeführtes Dokument
...............................................................
& : Mitglied der gleichen Patentfamilie, übereinstimmendes Dokument

EPO FORM 1503 03.82 (P04C03)

**ANHANG ZUM EUROPÄISCHEN RECHERCHENBERICHT
ÜBER DIE EUROPÄISCHE PATENTANMELDUNG NR.**

EP 08 01 5982

In diesem Anhang sind die Mitglieder der Patentfamilien der im obengenannten europäischen Recherchenbericht angeführten Patentdokumente angegeben.
Die Angaben über die Familienmitglieder entsprechen dem Stand der Datei des Europäischen Patentamts am
Diese Angaben dienen nur zur Unterrichtung und erfolgen ohne Gewähr.

22-12-2008

| Im Recherchenbericht angeführtes Patentdokument | Datum der Veröffentlichung | Mitglied(er) der Patentfamilie | Datum der Veröffentlichung |
|---|---|---|---|
| US 2007208243 A1 | 06-09-2007 | KEINE | |
| EP 1785085 A | 16-05-2007 | CA 2567434 A1<br>CN 1961821 A<br>EP 1785086 A2<br>JP 2007130482 A<br>US 2007151868 A1 | 12-05-2007<br>16-05-2007<br>16-05-2007<br>31-05-2007<br>05-07-2007 |
| EP 1733676 A | 20-12-2006 | CA 2550171 A1<br>JP 2006346454 A<br>US 2006287591 A1 | 17-12-2006<br>28-12-2006<br>21-12-2006 |
| US 4655880 A | 07-04-1987 | KEINE | |
| WO 2007147475 A | 27-12-2007 | KEINE | |
| US 2005059871 A1 | 17-03-2005 | US 2008033272 A1 | 07-02-2008 |

EPO FORM P0461

Für nähere Einzelheiten zu diesem Anhang : siehe Amtsblatt des Europäischen Patentamts, Nr.12/82

## IN DER BESCHREIBUNG AUFGEFÜHRTE DOKUMENTE

*Diese Liste der vom Anmelder aufgeführten Dokumente wurde ausschließlich zur Information des Lesers aufgenommen und ist nicht Bestandteil des europäischen Patentdokumentes. Sie wurde mit größter Sorgfalt zusammengestellt; das EPA übernimmt jedoch keinerlei Haftung für etwaige Fehler oder Auslassungen.*

**In der Beschreibung aufgeführte Patentdokumente**

- WO 2007147475 A
- US 4655880 A
- US 20050059871 A1
- US 20070151868 A1